# EUROPEAN PATENT SPECIFICATION

(11) **EP 1 686 184 B1**
(45) Date of publication and mention of the grant of the patent: **27.08.2008**
(21) Application number: 06113407.8
(22) Date of filing: 06.06.2002
(51) Int. Cl.: C12P 13/06, C12P 13/08

(54) **Process for the preparation of L-threonine using strains of the enterobacteriaceae family**
Verfahren zur Herstellung von L-Threonine unter Verwendung von Stämmen der Familie Enterobacteriaceae
Procédé pour la préparation de L-Threonine à l'aide de souches de la famille des enterobacteriaceae

(30) Priority: 11.07.2001 DE 10133667; 16.07.2001 US 305144 P
(43) Date of publication of application: 02.08.2006
(62) Divisional of application: 02738152.4
(73) Proprietor: Evonik Degussa GmbH, 45128 Essen (DE)
(72) Inventor: Siebelt, Nicole, 33775 Versmold (DE); Widawka, Petra, 33615 Bielefeld (DE); Farwick, Mike, 45130 Essen (DE)

(56) References cited:
- WO-A-98/04715
- DE-A- 10 116 518
- DE-A- 10 136 986
- BYRNE C R ET AL: "DNA SEQUENCES OF THE CYS-K REGIONS OF SALMONELLA-TYPHIMURIUM AND ESCHERICHIA-COLI AND LINKAGE OF THE CYS-K REGIONS TO PTS-H" JOURNAL OF BACTERIOLOGY, vol. 170, no. 1, 1988, pages 3150-3157, XP002385755 ISSN: 0021-9193
- DASSLER T ET AL: "IDENTIFICATION OF A MAJOR FACILITATOR PROTEIN FROM ESCHERICHIA COLI INVOLVED IN EFFLUX OF METABOLITES OF THE CYSTEINE PATHWAY" MOLECULAR MICROBIOLOGY, BLACKWELL SCIENTIFIC, OXFORD, GB, vol. 36, no. 5, June 2000 (2000-06), pages 1101-1112, XP000992212 ISSN: 0950-382X

## Description

### Field of the Invention

This invention relates to a process for the preparation of L-threonine, using strains of the Enterobacteriaceae family in which at least the cysK gene of the cysteine biosynthesis pathway (cysteine biosynthetic pathway) is enhanced.

### Prior Art

L-Amino acids, in particular L-threonine, are used in human medicine and in the pharmaceuticals industry, in the foodstuffs industry and very particularly in animal nutrition.

It is known to prepare L-amino acids by fermentation of strains of Enterobacteriaceae, in particular Escherichia coli (E. coli) and Serratia marcescens. Because of their great importance, work is constantly being undertaken to improve the preparation processes. Improvements to the process can relate to fermentation measures, such as e.g. stirring and supply of oxygen, or the composition of the nutrient media, such as e.g. the sugar concentration during the fermentation, or the working up to the product form, by e.g. ion exchange chromatography, or the intrinsic output properties of the microorganism itself.

Methods of mutagenesis, selection and mutant selection are used to improve the output properties of these microorganisms. Strains.which are resistant to antimetabolites, such as e.g. the threonine analogue α-amino-β-hydroxyvaleric acid (AHV), or are auxotrophic for metabolites of regulatory importance and produce L-amino acids, such as e.g. L-threonine, are obtained in this manner.

Methods of the recombinant DNA technique have also been employed for some years for improving the strain of strains of the Enterobacteriaceae family which produce L-amino acids, by amplifying individual amino acid biosynthesis genes and investigating the effect on the production.

### Objet of the Invention

The object of the invention is to provide new measures for improved fermentative preparation of L-amino acids, in particular L-threonine.

### Summary of the Invention

The invention provides a process for the fermentative preparation of L-threonine, using microorganisms of the Enterobacteriaceae family which in particular already produce L-threonine and in which the nucleotide sequence which code for the cysK gene of the cysteine biosynthesis pathway (cysteine biosynthetic pathway) is enhanced.

The process according to the invention for the preparation of amino acids comprises the following steps:
a) fermentation of the microorganisms of the Enterobacteriaceae family which produce the desired L-amino acid and in which the cysK gene of the cysteine biosynthesis pathway or nucleotide sequences which code for it is/are enhanced, in particular over-expressed,
b) concentration of the desired L-amino acid in the medium or in the cells of the microorganisms, and
c) isolation of the desired L-amino acid, constituents of the fermentation broth and/or the biomass in its entirety or portions (> 0 to 100%) thereof optionally remaining in the product.

### Detailed Description of the Invention

The use of endogenous genes is preferred. "Endogenous genes" or "endogenous nucleotide sequences" are understood as meaning the genes or nucleotide sequences present in the population of a species.

Where L-amino acids or amino acids are mentioned in the following, this means one or more amino acids, including their salts, chosen from the group consisting of L-asparagine, L-threonine, L-serine, L-glutamate, L-glycine, L-alanine, L-cysteine, L-valine, L-methionine, L-isoleucine, L-leucine, L-tyrosine, L-phenylalanine, L-histidine, L-lysine, L-tryptophan and L-arginine. L-Threonine is particularly preferred.

The term "enhancement" in this connection describes the increase in the intracellular activity or concentration of one or more enzymes or proteins in a microorganism which are coded by the corresponding DNA, for example by increasing the number of copies of the gene or genes, using a potent promoter or a gene or allele which codes for a corresponding enzyme or protein with a high activity, and optionally combining these measures.

By enhancement measures, in particular over-expression, the activity or concentration of the corresponding protein is in general increased by at least 10%, 25%, 50%, 75%, 100%, 150%, 200%, 300%, 400% or 500%, up to a maximum of 1000% or 2000%, based on that of the wild-type protein or the activity or concentration of the protein in the starting microorganism.

The process comprises carrying out the following steps:
a) fermentation of microorganisms of the Enterobacteriaceae family which produce L-threonine and in which the cysK gene of the cysteine biosynthesis pathway, or nucleotide sequences which code for the gene product of said gene, is (are) over-expressed by increasing the number of copies of said gene or nucleotide sequences and/or by using a potent promoter,
b) concentration of the L-threonine in the medium or in the cells of the microorganisms of the Enterobacteriaceae family, and
c) isolation of the L-threonine, constituents of the fermentation broth and/or the biomass in its entirety or portions (> 0 to 100 %) thereof optionally remaining in the product.

The microorganisms which the present invention provides can produce L-amino acids from glucose, sucrose, lactose, fructose, maltose, molasses, optionally starch, optionally cellulose or from glycerol and ethanol. They are representatives of the Enterobacteriaceae family chosen from the genera Escherichia, Erwinia, Providencia and Serratia. The genera Escherichia and Serratia are preferred. Of the genus Escherichia the species Escherichia coli and of the genus Serratia the species Serratia marcescens are to be mentioned in particular.

Suitable strains, which produce L-threonine in particular, of the genus Escherichia, in particular of the species Escherichia coli, are, for example
Escherichia coli TF427
Escherichia coli H4578
Escherichia coli KY10935
Escherichia coli VNIIgenetika MG442
Escherichia coli VNIIgenetika M1
Escherichia coli VNIIgenetika 472T23
Escherichia coli BKIIM B-3996
Escherichia coli kat 13
Escherichia coli KCCM-10132

Suitable L-threonine-producing strains of the genus Serratia, in particular of the species Serratia marcescens, are, for example
Serratia marcescens HNr21
Serratia marcescens TLr156
Serratia marcescens T2000

Strains from the Enterobacteriaceae family which produce L-threonine preferably have, inter alia, one or more genetic or phenotypic features chosen from the group consisting of: resistance to α-amino-β-hydroxyvaleric acid, resistance to thialysine, resistance to ethionine, resistance to α-methylserine, resistance to diaminosuccinic acid, resistance to α-aminobutyric acid, resistance to borrelidin, resistance to rifampicin, resistance to valine analogues, such as, for example, valine hydroxamate, resistance to purine analogues, such as, for example, 6-dimethylaminopurine, a need for L-methionine, optionally a partial and compensable need for L-isoleucine, a need for meso-diaminopimelic acid, auxotrophy in respect of threonine-containing dipeptides, resistance to L-threonine, resistance to L-homoserine, resistance to L-lysine, resistance to L-methionine, resistance to L-glutamic acid, resistance to L-aspartate, resistance to L-leucine, resistance to L-phenylalanine, resistance to L-serine, resistance to L-cysteine, resistance to L-valine, sensitivity to fluoropyruvate, defective threonine dehydrogenase, optionally an ability for sucrose utilization, enhancement of the threonine operon, enhancement of homoserine dehydrogenase I-aspartate kinase I, preferably of the feed back resistant form, enhancement of homoserine kinase, enhancement of threonine synthase, enhancement of aspartate kinase, optionally of the feed back resistant form, enhancement of aspartate semialdehyde dehydrogenase, enhancement of phosphoenol pyruvate carboxylase, optionally of the feed back resistant form, enhancement of phosphoenol pyruvate synthase, enhancement of transhydrogenase, enhancement of the RhtB gene product, enhancement of the RhtC gene product, enhancement of the YfiK gene product, enhancement of a pyruvate carboxylase, and attenuation of acetic acid formation.

It has been found that microorganisms of the Enterobacteriaceae family produce L-amino acids, in particular L-threonine, in an improved manner after enhancement, in particular over-expression, of at least one or more of the genes of the cysteine biosynthesis pathway chosen from the group consisting of cysG, cysB, cysZ, cysK, cysM, cysA, cysW, cysU, cysP, cysD, cysN, cysC, cysJ, cysI, cysH, cysE and sbp.

The nucleotide sequences of the genes of Escherichia coli belong to the prior art (See following text references) and can also be found in the genome sequence of Escherichia coli published by Blattner et al. (Science 277: 1453 - 1462 (1997)). The genes and activities of the cysteine biosynthesis pathway (cysteine biosynthetic pathway) are also described in summary form in Kredich (In: Neidhardt (ed), Escherichia coli and Salmonella, American Society for Microbiology, Washington, D.C., USA: 514-527 (1996)).

| cysG gene: | |
|---|---|
| Description: | Uroporphyrinogen III C methyl-transferase; precorrin-2 oxidase; ferrochelatase |
| EC No.: | 2.1.1.107; 1.-.-.-; 4.99.1.- |

| | |
|---|---|
| Reference: | Peakman et al.; European Journal of Biochemistry 191(2): 315-323 (1990) Macdonald and Cole; Molecular and General Genetics 200(2): 328-334 (1985) Warren et al.; Biochemical Journal 265(3):725-729 (1990) Spencer et al.; FEBS Letters 335(1): 57-60 (1993) |
| Accession No.: | AE000412 |

| cysB gene: | |
|---|---|
| Description: | Positive regulator of the cys regulon, transcription activator |
| Reference: | Ostrowski et al.; Journal of Biological Chemistry 262(13): 5999-6005 (1987) Mascarenhas and Yudkin; Molecular and General Genetics 177(3): 535-539 (1980) Lochowska et al.; Journal of Biological Chemistry 276(3): 2098-2107 (2001) |
| Accession No.: | AE000225 |

| cysZ gene: | |
|---|---|
| Description: | Sulfate transporter |
| Reference: | Byrne et al.; Journal of Bacteriology 170(7): 3150-3157 (1988) |
| Accession No.: | AE000329 |

| cysK gene: | |
|---|---|
| Description: | Cysteine synthase A, O-acetylserine (thiol)-lyase A |
| EC No.: | 4.2.99.8 |
| Reference: | Byrne et al.; Journal of Bacteriology 170(7): 3150-3157 (1988) Boronat et al.; Journal of General Microbiology 130: 673- 685 (1984) Levy and Danchin; Molecular Microbiology 2(6): 777-783 (1988) |
| Accession No.: | AE000329 |
| Alternative gene name: | cysZ |

| cysM gene: | |
|---|---|
| Description: | Cysteine synthase B, O-acetylserine (thiol)-lyase B |
| EC No.: | 4.2.99.8 |
| Reference: | Sirko et al.; Journal of Bacteriology 172(6): 3351-3357 (1990) Sirko et al.; Journal of General Microbiology 133: 2719- 2725 (1987) |
| Accession No.: | AE000329 |

| cysA gene: | |
|---|---|
| Description: | ATP-binding protein of the sulfate transport system |
| Reference: | Sirko et al.; Journal of Bacteriology 172(6): 3351-3357 (1990) Sirko et al.; Journal of General Microbiology 133: 2719- 2725 (1987) |
| Accession No.: | AE000329 |

| cysW gene: | |
|---|---|
| Description: | Membrane-bound sulfate transport protein |
| Reference: | Sirko et al.; Journal of Bacteriology 172(6): 3351-3357 (1990) |
| Accession No.: | AE000329, AE000330 |

| cysU gene: | |
|---|---|
| Description: | Permease protein of the sulfate transport system |
| Reference: | Sirko et al.; Journal of Bacteriology 172(6): 3351-3357 (1990) Hryniewicz et al.; Journal of Bacteriology 172(6): 3358-3366 (1990) |
| Accession No.: | AE000330 |
| Alternative gene name: | cysT |

| cysP gene: | |
|---|---|
| Description: | Periplasmic thiosulfate-binding protein |

| | |
|---|---|
| Reference: | Hryniewicz et al.; Journal of Bacteriology 172(6): 3358-3366 (1990) Sirko et al.; Journal of Bacteriology 177(14): 4134-4136 (1995) |
| Accession No.: | AE000330 |

| cysD gene: | |
|---|---|
| Description: | Sub-unit 2 of ATP sulfurylase (ATP:sulfate adenylyl-transferase) |
| EC No.: | 2.7.7.4 |
| Reference: | Leyh et al.; Journal of Biological Chemistry 267(15): 10405-10410 (1992) Leyh et al.; Journal of Biological Chemistry 263(5): 2409-2416 (1988) |
| Accession No.: | AE000358 |

| cysN gene: | |
|---|---|
| Description: | Sub-unit 1 of ATP sulfurylase (ATP:sulfate adenylyl-transferase) |
| EC No.: | 2.7.7.4 |
| Reference: | Leyh et al.; Journal of Biological Chemistry 267(15): 10405-10410 (1992) Leyh et al.; Journal of Biological Chemistry 263(5): 2409-2416 (1988) Leyh and Suo; Journal of Biological Chemistry 267(1): 542-545 (1992) |
| Accession No.: | AE000358 |

| cysC gene: | |
|---|---|
| Description: | Adenylyl sulfate kinase (APS kinase) |
| EC No.: | 2.7.1.25 |
| Reference: | Leyh et al.; Journal of Biological Chemistry 267(15): 10405-10410 (1992) Leyh et al.; Journal of Biological Chemistry 263(5): 2409-2416 (1988) |
| Accession No.: | AE000358 |

| cysJ gene: | |
|---|---|
| Description: | Flavoprotein of NADPH sulfite reductase |
| EC No.: | 1.8.1.2 |
| Reference: | Ostrowski et al.; Journal of Biological Chemistry 264(27): 15796-15808 (1989) Li et al.; Gene 53(2-3): 227-234 (1987) Gaudu and Fontecave; European Journal of Biochemistry 226(2): 459-463 (1994) Eschenbrenner et al.; Journal of Biological Chemistry 270(35): 20550-20555 (1995) |
| Accession No.: | AE000360 |
| Alternative gene name: | cysP |

| cysI gene: | |
|---|---|
| Description: | Haemoprotein of NADPH sulfite reductase |
| EC No.: | 1.8.1.2 |
| Reference: | Ostrowski et al.; Journal of Biological Chemistry 264(26): 15726-15737 (1989) Li et al.; Gene 53(2-3): 227-234 (1987) Gaudu and Fontecave; European Journal of Biochemistry 226(2): 459-463 (1994) |
| Accession No.: | AE000360 |
| Alternative gene name: | cysQ |

| cysH gene: | |
|---|---|
| Description: | Phosphoadenosine phosphosulfate reductase (PAPS reductase) |
| EC No.: | 1.8.99.4 |
| Reference: | Ostrowski et al.; Journal of Biological Chemistry 264(26): 15726-15737 (1989) Krone et al.; Molecular and General Genetics 225(2): 314-319 (1991) Li et al.; Gene 53(2-3): 227-234 (1987) Berendt et al.; European Journal of Biochemistry 233(1): 347-356 (1995) |
| Accession No.: | AE000360 |

| cysE gene: | |
|---|---|
| Description: | Serine acetyl-transferase |
| EC No.: | 2.3.1.30 |
| Reference: | Denk and Böck; Journal of General Microbiology 133, 515-25 (1987) |
| Accession No.: | AE000438 |

| sbp gene: | |
|---|---|
| Description: | Periplasmic sulfate-binding protein |
| Reference: | Hellinga and Evans, European Journal of Biochemistry 149(2): 363-373 (1985) Sirko et al.; Journal of Bacteriology 177(14): 4134-4136 (1995) Jacobson et al.; Journal of Biological Chemistry 266(8): 5220-5225 (1991) |
| Accession No.: | AE000466 |

The nucleic acid sequences can be found in the databanks of the National Center for Biotechnology Information (NCBI) of the National Library of Medicine (Bethesda, MD, USA), the nucleotide sequence databank of the European Molecular Biologies Laboratories (EMBL, Heidelberg, Germany or Cambridge, UK) or the DNA databank of Japan (DDBJ, Mishima, Japan) .

The genes described in the text references mentioned can be used according to the invention. Alleles of the genes which result from the degeneracy of the genetic code or due to "sense mutations" of neutral function can furthermore be used.

To achieve an enhancement, for example, expression of the genes or the catalytic properties of the proteins can be increased. The two measures can optionally be combined.

To achieve an over-expression, the number of copies of the corresponding genes can be increased, or the promoter and regulation region or the ribosome binding site upstream of the structural gene can be mutated. Expression cassettes which are incorporated upstream of the structural gene act in the same way. By inducible promoters, it is additionally possible to increase the expression in the course of fermentative L-threonine production. The expression is likewise improved by measures to prolong the life of the m-RNA. Furthermore, the enzyme activity is also enhanced by preventing the degradation of the enzyme protein. The genes or gene constructs can either be present in plasmids with a varying number of copies, or can be integrated and amplified in the chromosome. Alternatively, an over-expression of the genes in question can furthermore be achieved by changing the composition of the media and the culture procedure.

Instructions in this context can be found by the expert, inter alia, in Chang and Cohen (Journal of Bacteriology 134: 1141-1156 (1978)), in Hartley and Gregori (Gene 13: 347-353 (1981)), in Amann and Brosius (Gene 40: 183-190 (1985)), in de Broer et al. (Proceedings of the National Academy of Sciences of the United States of America 80: 21-25 (1983)), in LaVallie et al. (BIO/TECHNOLOGY 11: 187-193 (1993)), in PCT/US97/13359, in Llosa et al. (Plasmid 26: 222-224 (1991)), in Quandt and Klipp (Gene 80: 161-169 (1989)), in Hamilton (Journal of Bacteriology 171: 4617-4622 (1989)), in Jensen and Hammer (Biotechnology and Bioengineering 58: 191-195 (1998)) and in known textbooks of genetics and molecular biology.

Plasmid vectors which are capable of replication in Enterobacteriaceae, such as e.g. cloning vectors derived from pACYC184 (Bartolomé et al.; Gene 102: 75-78 (1991)), pTrc99A (Amann et al.; (Gene 69: 301-315 (1988)) or pSC101 derivatives (Vocke and Bastia, Proceedings of the National Academy of Sciences USA 80 (21): 6557-6561 (1983)) can be used. A strain transformed with a plasmid vector, where the plasmid vector carries the cysK or nucleotide sequences which code for it, can be employed in a process according to the invention.

It is also possible to transfer mutations which affect the expression of the particular gene into various strains by sequence exchange (Hamilton et al. (Journal of Bacteriology 171: 4617 - 4622 (1989)), conjugation or transduction.

It may furthermore be advantageous for the production of L-amino acids, in particular L-threonine, with strains of the Enterobacteriaceae family to enhance one or more enzymes of the known threonine biosynthesis pathway or enzymes of anaplerotic metabolism or enzymes for the production of reduced nicotinamide adenine dinucleotide phosphate or enzymes of glycolysis or PTS enzymes, in addition to enhancement of the cysK gene of the cysteine biosynthesis pathway.

Thus, for example, at the same time one or more of the genes chosen from the group consisting of
- the thrABC operon which codes for aspartate kinase, homoserine dehydrogenase, homoserine kinase and threonine synthase (US-A-4,278,765),
- the pyc gene of Corynebacterium glutamicum which codes for pyruvate carboxylase (WO 99/18228),
- the pps gene which codes for phosphoenol pyruvate synthase (Molecular and General Genetics 231: 332-336 (1992)),
- the ppc gene which codes for phosphoenol pyruvate carboxylase (Gene 31: 279-283 (1984)),
- the pntA and pntB genes which code for transhydrogenase (European Journal of Biochemistry 158: 647-653 (1986)),
- the rhtB gene which imparts homoserine resistance (EP-A-0 994 190),
- the mqo gene which codes for malate:quinone oxidoreductase (WO 02/06459),
- the rhtC gene which imparts threonine resistance (EP-A-1 013 765),
- the thrE gene of Corynebacterium glutamicum which codes for threonine export (WO 01/92545),
- the gdhA gene which codes for glutamate dehydrogenase (Nucleic Acids Research 11: 5257-5266 (1983); Gene 23: 199-209 (1983))
- the hns gene which codes for the DNA-binding protein HLP-II (Molecular and General Genetics 212: 199-202 (1988)),
- the pgm gene which codes for phosphoglucomutase (Journal of Bacteriology 176: 5847-5851 (1994)),
- the fba gene which codes for fructose biphosphate aldolase (Biochemical Journal 257: 529-534 (1989)),
- the ptsH gene of the ptsHIcrr operon which codes for the phosphohistidine protein hexose phosphotransferase of the phosphotransferase system PTS (Journal of Biological Chemistry 262: 16241-16253 (1987)),
- the ptsI gene of the ptsHIcrr operon which codes for enzyme I of the phosphotransferase system PTS (Journal of Biological Chemistry 262: 16241-16253 (1987)),
- the crr gene of the ptsHIcrr operon which codes for the glucose-specific IIA component of the phosphotransferase system PTS (Journal of Biological Chemistry 262: 16241-16253 (1987)),
- the ptsG gene which codes for the glucose-specific IIBC component (Journal of Biological Chemistry 261: 16398-16403 (1986)),
- the lrp gene which codes for the regulator of the leucine regulon (Journal of Biological Chemistry 266: 10768-10774 (1991)),
- the mopB gene which codes for 10 Kd chaperone (Journal of Biological Chemistry 261: 12414-12419 (1986)) and is also known by the name groES,
- the ahpC gene of the ahpCF operon which codes for the small sub-unit of alkyl hydroperoxide reductase (Proceedings of the National Academy of Sciences USA 92: 7617-7621 (1995))
- the ahpF gene of the ahpCF operon which codes for the large sub-unit of alkyl hydroperoxide reductase (Proceedings of the National Academy of Sciences USA 92: 7617-7621 (1995))
can be over-expressed.

It may furthermore be advantageous for the production of L-threonine, in addition to enhancement of the cysK gene , for one or more of the genes chosen from the group consisting of
- the tdh gene which codes for threonine dehydrogenase (Ravnikar and Somerville (Journal of Bacteriology 169: 4716-4721 (1987)),
- the mdh gene which codes for malate dehydrogenase (E.C. 1.1.1.37) (Vogel et al. (Archives in Microbiology 149: 36-42 (1987)),
- the gene product of the open reading frame (orf) yjfA (Accession Number AAC77180 of the National Center for Biotechnology Information (NCBI, Bethesda, MD, USA)),
- the gene product of the open reading frame (orf) ytfP (Accession Number AAC77179 of the National Center for Biotechnology Information (NCBI, Bethesda, MD, USA)),
- the pckA gene which codes for the enzyme phosphoenol pyruvate carboxykinase (Medina et al. (Journal of Bacteriology 172: 7151-7156 (1990)),
- the poxB gene which codes for pyruvate oxidase (Grabau and Cronan (Nucleic Acids Research 14 (13), 5449-5460 (1986)),
- the aceA gene which codes for the enzyme isocitrate lyase (Matsuoko and McFadden (Journal of Bacteriology 170, 4528-4536 (1988)),
- the dgsA gene which codes for the DgsA regulator of the phosphotransferase system (Hosono et al. (Bioscience, Biotechnology and Biochemistry 59: 256-251 (1995)) and is also known under the name of the mlc gene,
- the fruR gene which codes for the fructose repressor (Jahreis et al. (Molecular and General Genetics 226: 332-336 (1991)) and is also known by the name of the cra gene, and
- the rpoS gene which codes for the sigma³⁸ factor (WO 01/05939) and is also known under the name of the katF gene,
to be eliminated or for the expression thereof to be reduced.

The term "attenuation" in this connection describes the reduction or elimination of the intracellular activity or concentration of one or more enzymes or proteins in a microorganism which are coded by the corresponding DNA, for example by using a weak promoter or a gene or allele which codes for a corresponding enzyme or protein with a low activity or inactivates the corresponding enzyme or protein or gene, and optionally combining these measures.

By attenuation measures, the activity or concentration of the corresponding protein is in general reduced to 0 to 75%, 0 to 50%, 0 to 25%, 0 to 10% or 0 to 5% of the activity or concentration of the wild-type protein or of the activity or concentration of the protein in the starting microorganism.

It may furthermore be advantageous for the production of L-amino acids, in particular L-threonine, in addition to enhancement of the cysK gene of the cysteine biosynthesis pathway , to eliminate undesirable side reactions (Nakayama: "Breeding of Amino Acid Producing Microorganisms", in: Overproduction of Microbial Products, Krumphanzl, Sikyta, Vanek (eds.), Academic Press, London, UK, 1982).

The microorganisms produced according to the invention can be cultured in the batch process (batch culture), the fed batch (feed process) or the repeated fed batch process (repetitive feed process). A summary of known culture methods is described in the textbook by Chmiel (Bioprozesstechnik 1. Einführung in die Bioverfahrenstechnik [Bioprocess Technology 1. Introduction to Bioprocess Technology (Gustav Fischer Verlag, Stuttgart, 1991)) or in the textbook by Storhas (Bioreaktoren und periphere Einrichtungen [Bioreactors and Peripheral Equipment] (Vieweg Verlag, Braunschweig/Wiesbaden, 1994)).

The culture medium to be used must meet the requirements of the particular strains in a suitable manner. Descriptions of culture media for various microorganisms are contained in the handbook "Manual of Methods for General Bacteriology" of the American Society for Bacteriology (Washington D.C., USA, 1981).

Sugars and carbohydrates, such as e.g. glucose, sucrose, lactose, fructose, maltose, molasses, starch and optionally cellulose, oils and fats, such as e.g. soya oil, sunflower oil, groundnut oil and coconut fat, fatty acids, such as e.g. palmitic acid, stearic acid and linoleic acid, alcohols, such as e.g. glycerol and ethanol, and organic acids, such as e.g. acetic acid, can be used as the source of carbon. These substances can be used individually or as a mixture.

Organic nitrogen-containing compounds, such as peptones, yeast extract, meat extract, malt extract, corn steep liquor, soya bean flour and urea, or inorganic compounds, such as ammonium sulfate, ammonium chloride, ammonium phosphate, ammonium carbonate and ammonium nitrate, can be used as the source of nitrogen. The sources of nitrogen can be used individually or as a mixture.

Phosphoric acid, potassium dihydrogen phosphate or dipotassium hydrogen phosphate or the corresponding sodium-containing salts can be used as the source of phosphorus. The culture medium must furthermore comprise salts of metals, such as e.g. magnesium sulfate or iron sulfate, which are necessary for growth. Finally, essential growth substances, such as amino acids and vitamins, can be employed in addition to the above-mentioned substances. Suitable precursors can moreover be added to the culture medium. The starting substances mentioned can be added to the culture in the form of a single batch, or can be fed in during the culture in a suitable manner.

Basic compounds, such as sodium hydroxide, potassium hydroxide, ammonia or aqueous ammonia, or acid compounds, such as phosphoric acid or sulfuric acid, can be employed in a suitable manner to control the pH of the culture. Antifoams, such as e.g. fatty acid polyglycol esters, can be employed to control the development of foam. Suitable substances having a selective action, e.g. antibiotics, can be added to the medium to maintain the stability of plasmids. To maintain aerobic conditions, oxygen or oxygen-containing gas mixtures, such as e.g. air, are introduced into the culture. The temperature of the culture is usually 25°C to 45°C, and preferably 30°C to 40°C. Culturing is continued until a maximum of L-amino acids or L-threonine has formed. This target is usually reached within 10 hours to 160 hours.

The analysis of L-amino acids can be carried out by anion exchange chromatography with subsequent ninhydrin derivation, as described by Spackman et al. (Analytical Chemistry 30: 1190-1206 (1958)), or it can take place by reversed phase HPLC as described by Lindroth et al. (Analytical Chemistry 51: 1167-1174 (1979)).

The process according to the invention is used for the fermentative preparation of L-threonine.

The minimal (M9) and complete media (LB) for Escherichia coli used are described by J.H. Miller (A short course in bacterial genetics (1992), Cold Spring Harbor Laboratory Press). The isolation of plasmid DNA from Escherichia coli and all techniques of restriction, ligation, Klenow and alkaline phosphatase treatment are carried out by the method of Sambrook et al. (Molecular cloning - A Laboratory Manual (1989) Cold Spring Harbor Laboratory Press). Unless described otherwise, the transformation of Escherichia coli is carried out by the method of Chung et al. (Proceedings of the National Academy of Sciences of the United States of America (1989) 86: 2172-2175).

The incubation temperature for the preparation of strains and transformants is 37°C.

### Example 1 (not part of the invention)

### Preparation of L-threonine using the cysB gene

### 1a) Construction of the expression plasmid pTrc99AcysB

The cysB gene from E. coli K12 is amplified using the polymerase chain reaction (PCR) and synthetic oligonucleotides. Starting from the nucleotide sequence of the cysB gene in E. coli K12 MG1655 (Accession Number AE000225, Blattner et al. (Science 277: 1453-1462 (1997)), PCR primers are synthesized (MWG Biotech, Ebersberg, Germany):
cysB1: 5' - GCGTCTAAGTGGATGGTTTAAC - 3' (SEQ ID No. 1)
cysB2: 5' - GGTGCCGAAAATAACGCAAG - 3' (SEQ ID No. 2)

The chromosomal E. coli K12 MG1655 DNA employed for the PCR is isolated according to the manufacturer,s instructions with "Qiagen Genomic-tips 100/G" (QIAGEN, Hilden, Germany). A DNA fragment approx. 1000 bp in size can be amplified with the specific primers under standard PCR conditions (Innis et al. (1990) PCR Protocols. A Guide to Methods and Applications, Academic Press) with Pfu-DNA polymerase (Promega Corporation, Madison, USA). The PCR product is ligated according to the manufacturer's instructions with the vector pCR-Blunt II-TOPO (Zero Blunt TOPO PCR Cloning Kit, Invitrogen, Groningen, The Netherlands) and transformed into the E. coli strain TOP10.

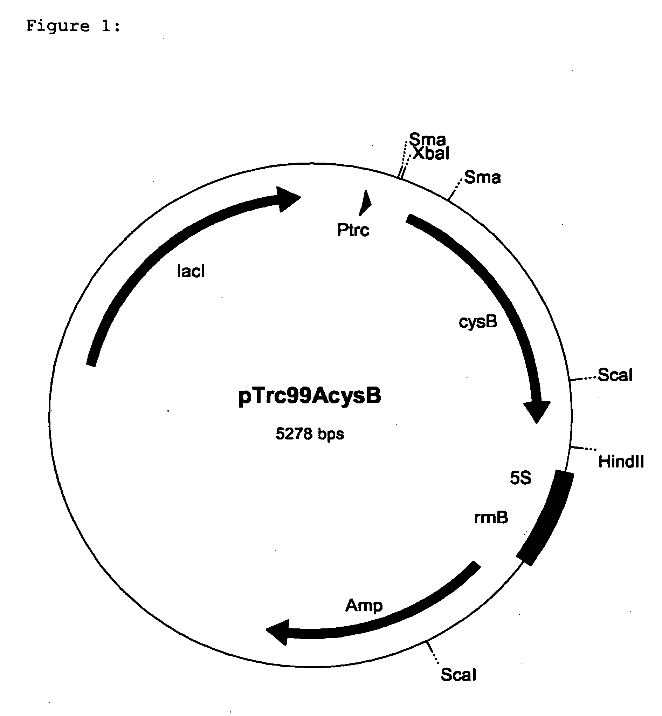

Selection of plasmid-carrying cells takes place on LB agar, to which 50 µg/ml kanamycin are added. After isolation of the plasmid DNA, the vector pCR-Blunt II-TOPO-cysB is cleaved with the restriction enzymes HindIII and XbaI and, after separation in 0.8% agarose gel, the cysB fragment is isolated with the aid of the QIAquick Gel Extraction Kit (QIAGEN, Hilden, Germany). The vector pTrc99A (Pharmacia Biotech, Uppsala, Sweden) is cleaved with the enzymes HindIII and XbaI and ligation is carried out with the cysB fragment isolated. The E. coli strain XL1-Blue MRF' (Stratagene, La Jolla, USA) is transformed with the ligation batch and plasmid-carrying cells are selected on LB agar, to which 50 µg/ml ampicillin are added. Successful cloning can be demonstrated after plasmid DNA isolation by control cleavage with the enzymes ScaI and SmaI. The plasmid is called pTrc99AcysB (Figure 1).

### 1b) Preparation of L-threonine with the strain MG442/pTrc99AcysB

The L-threonine-producing E. coli strain MG442 is described in the patent specification US-A- 4,278,765 and deposited as CMIM B-1628 at the Russian National Collection for Industrial Microorganisms (VKPM, Moscow, Russia).

The strain MG442 is transformed with the expression plasmid pTrc99AcysB described in example Ia and with the vector pTrc99A and plasmid-carrying cells are selected on LB agar with 50 µg/ml ampicillin. The strains MG442/pTrc99AcysB and MG442/pTrc99A are formed in this manner. Selected individual colonies are then multiplied further on minimal medium with the following composition: 3.5 g/l Na₂HPO₄*2H₂O, 1.5 g/l KH₂PO₄, 1 g/l NH₄Cl, 0.1 g/l MgSO₄*7H₂O, 2 g/l glucose, 20 g/l agar, 50 mg/l ampicillin. The formation of L-threonine is checked in batch cultures of 10 ml contained in 100 ml conical flasks. For this, 10 ml of preculture medium of the following composition: 2 g/l yeast extract, 10 g/l (NH₄)₂SO₄, 1 g/l KH₂PO₄, 0.5 g/l MgSO₄*7H₂O, 15 g/l CaCO₃, 20 g/l glucose, 50 mg/l ampicillin are inoculated and the batch is incubated for 16 hours at 37°C and 180 rpm on an ESR incubator from Kühner AG (Birsfelden, Switzerland).

250 µl portions of this preculture are transinoculated into 10 ml of production medium (25 g/l (NH₄)₂SO₄, 2 g/l KH₂PO₄, 1 g/l MgSO₄*7H₂O, 0.03 g/l FeSO₄*7H₂O, 0.018 g/l MnSO₄*1H₂O, 30 g/l CaCO₃, 20 g/l glucose, 50 mg/l ampicillin) and the batch is incubated for 48 hours at 37°C. For complete induction of the expression of the cysB gene, 100 mg/l isopropyl β-D-thiogalactopyranoside (IPTG) are added in parallel batches. The formation of L-threonine by the starting strain MG442 is investigated in the same manner, but no addition of ampicillin to the medium takes place. After the incubation the optical density (OD) of the culture suspension is determined with an LP2W photometer from Dr. Lange (Düsseldorf, Germany) at a measurement wavelength of 660 nm.

The concentration of L-threonine formed is then determined in the sterile-filtered culture supernatant with an amino acid analyzer from Eppendorf-BioTronik (Hamburg, Germany) by ion exchange chromatography and post-column reaction with ninhydrin detection.

The result of the experiment is shown in Table 1.

**Table 1**

| Strain | Additives | OD (660 nm) | L-Threonine g/l |
|---|---|---|---|
| MG442 | - | 5.6 | 1.4 |
| MG442/pTrc99A | - | 3.8 | 1.3 |
| MG442/pTrc99AcysB | - | 4.4 | 1.7 |
| MG442/pTrc99AcysB | IPTG | 5.4 | 2.0 |

### Example 2

### Preparation of L-threonine using the cysK gene

### 2a) Construction of the expression plasmid pTrc99AcysK

The cysK gene from E. coli K12 is amplified using the polymerase chain reaction (PCR) and synthetic oligonucleotides. Starting from the nucleotide sequence of the cysK gene in E. coli K12 MG1655 (Accession Number AE000329, Blattner et al. (Science 277: 1453-1462 (1997)), PCR primers are synthesized (MWG Biotech, Ebersberg, Germany):
cysK1: 5' - CAGTTAAGGACAGGCCATGAG - 3' (SEQ ID No. 3)
cysK2: 5' - GCTGGCATTACTGTTGCAATTC - 3' (SEQ ID No. 4)

The chromosomal E. coli K12 MG1655 DNA employed for the PCR is isolated according to the manufacturer,s instructions with "Qiagen Genomic-tips 100/G" (QIAGEN, Hilden, Germany). A DNA fragment approx. 1000 bp in size can be amplified with the specific primers under standard PCR conditions (Innis et al. (1990) PCR Protocols. A Guide to Methods and Applications, Academic Press) with Pfu-DNA polymerase (Promega Corporation, Madison, USA). The PCR product is ligated according to the manufacturer's instructions with the vector pCR-Blunt II-TOPO (Zero Blunt TOPO PCR Cloning Kit, Invitrogen, Groningen, The Netherlands) and transformed into the E. coli strain TOP10.

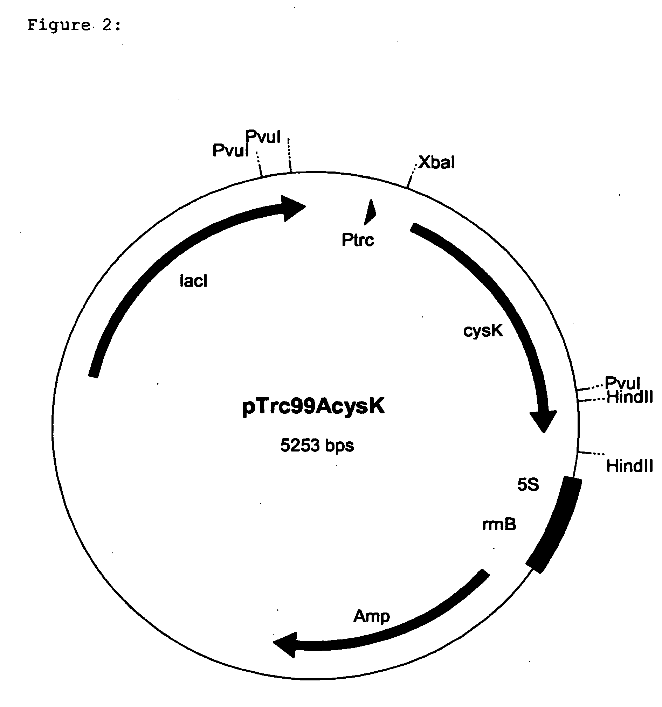

Selection of plasmid-carrying cells takes place on LB agar, to which 50 µg/ml kanamycin are added. After isolation of the plasmid DNA, the vector pCR-Blunt II-TOPO-cysK is cleaved with the restriction enzymes SpeI and XbaI and, after separation in 0.8% agarose gel, the cysK fragment is isolated with the aid of the QIAquick Gel Extraction Kit (QIAGEN, Hilden, Germany). The vector pTrc99A (Pharmacia Biotech, Uppsala, Sweden) is cleaved with the enzyme XbaI and ligation is carried out with the cysK fragment isolated. The E. coli strain XL1-Blue MRF' (Stratagene, La Jolla, USA) is transformed with the ligation batch and plasmid-carrying cells are selected on LB agar, to which 50 µg/ml ampicillin are added. Successful cloning can be demonstrated after plasmid DNA isolation by control cleavage with the enzymes HindIII and PvuII. The plasmid is called pTrc99AcysK (Figure 2).

### 2b) Preparation of L-threonine with the strain MG442/pTrc99AcysK

The L-threonine-producing E. coli strain MG442 is described in the patent specification US-A- 4,278,765 and deposited as CMIM B-1628 at the Russian National Collection for Industrial Microorganisms (VKPM, Moscow, Russia).

The strain MG442 is transformed with the expression plasmid pTrc99AcysK described in example 2a and with the vector pTrc99A and plasmid-carrying cells are selected on LB agar with 50 µg/ml ampicillin. The strains MG442/pTrc99AcysK and MG442/pTrc99A are formed in this manner. Selected individual colonies are then multiplied further on minimal medium with the following composition: 3.5 g/l Na₂HPO₄*2H₂O, 1.5 g/l KH₂PO₄, 1 g/l NH₄Cl, 0.1 g/l MgSO₄*7H₂O, 2 g/l glucose, 20 g/l agar, 50 mg/l ampicillin. The formation of L-threonine is checked in batch cultures of 10 ml contained in 100 ml conical flasks. For this, 10 ml of preculture medium of the following composition: 2 g/l yeast extract, 10 g/l (NH₄)₂SO₄, 1 g/l KH₂PO₄, 0.5 g/l MgSO₄*7H₂O, 15 g/l CaCO₃, 20 g/l glucose, 50 mg/l ampicillin are inoculated and the batch is incubated for 16 hours at 37°C and 180 rpm on an ESR incubator from Kuhner AG (Birsfelden, Switzerland).

250 µl portions of this preculture are transinoculated into 10 ml of production medium (25 g/l (NH₄)₂SO₄, 2 g/l KH₂PO₄, 1 g/l MgSO₄*7H₂O, 0.03 g/l FeSO₄*7H₂O, 0.018 g/l MnSO₄*1H₂O, 30 g/l CaCO₃, 20 g/l glucose, 50 mg/l ampicillin) and the batch is incubated for 48 hours at 37°C. The formation of L-threonine by the starting strain MG442 is investigated in the same manner, but no addition of ampicillin to the medium takes place. After the incubation the optical density (OD) of the culture suspension is determined with an LP2W photometer from Dr. Lange (Düsseldorf, Germany) at a measurement wavelength of 660 nm.

The concentration of L-threonine formed is then determined in the sterile-filtered culture supernatant with an amino acid analyzer from Eppendorf-BioTronik (Hamburg, Germany) by ion exchange chromatography and post-column reaction with ninhydrin detection.

The result of the experiment is shown in Table 2.

**Table 2**

| Strain | OD (660 nm) | L-Threonine g/l |
|---|---|---|
| MG442 | 5.6 | 1.4 |
| MG442/pTrc99A | 3.8 | 1.3 |
| MG442/pTrc99AcysK | 5.6 | 2.1 |

### Example 3 (not part of the invention)

### Preparation of L-threonine using the cysM gene

### 3a) Construction of the expression plasmid pTrc99AcysM

The cysM gene from E. coli K12 is amplified using the polymerase chain reaction (PCR) and synthetic oligonucleotides. Starting from the nucleotide sequence of the cysM gene in E. coli K12 MG1655 (Accession Number AE000329, Blattner et al. (Science 277: 1453-1462 (1997)), PCR primers are synthesized (MWG Biotech, Ebersberg, Germany). The sequences of the primers are modified such that recognition sites for restriction enzymes are formed. The recognition sequence for XbaI is chosen for the cysM1 primer and the recognition sequence for HindIII for the cysM2 primer, which are marked by underlining in the nucleotide sequence shown below:
cysM1: 5' - CGCATCAGTCTAGACCACGTTAGGATAG - 3' (SEQ ID No. 5)
cysM2: 5' - CATCAGTCTCCGAAGCTTTTAATCC - 3' (SEQ ID No. 6)

The chromosomal E. coli K12 MG1655 DNA employed for the PCR is isolated according to the manufacturer's instructions with "Qiagen Genomic-tips 100/G" (QIAGEN, Hilden, Germany). A DNA fragment approx. 950 bp in size can be amplified with the specific primers under standard PCR conditions (Innis et al. (1990) PCR Protocols. A Guide to Methods and Applications, Academic Press) with Pfu-DNA polymerase (Promega Corporation, Madison, USA). The PCR product is ligated according to the manufacturer's instructions with the vector pCR-Blunt II-TOPO (Zero Blunt TOPO PCR Cloning Kit, Invitrogen, Groningen, The Netherlands) and transformed into the E. coli strain TOP10.

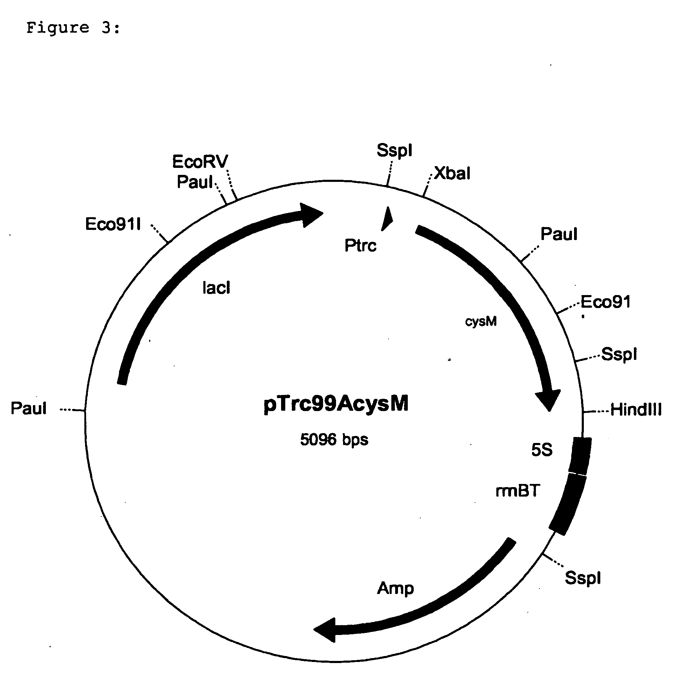

Selection of plasmid-carrying cells takes place on LB agar, to which 50 µg/ml kanamycin are added. After isolation of the plasmid DNA, the vector pCR-Blunt II-TOPO-cysM is cleaved with the restriction enzymes HindIII and XbaI and, after separation in 0.8% agarose gel, the cysM fragment is isolated with the aid of the QIAquick Gel Extraction Kit (QIAGEN, Hilden, Germany). The vector pTrc99A (Pharmacia Biotech, Uppsala, Sweden) is cleaved with the enzymes HindIII and XbaI and ligation is carried out with the cysM fragment isolated. The E. coli strain XL1-Blue MRF' (Stratagene, La Jolla, USA) is transformed with the ligation batch and plasmid-carrying cells are selected on LB agar, to which 50 µg/ml ampicillin are added. Successful cloning can be demonstrated after plasmid DNA isolation by control cleavage with the enzymes EcoRV, Eco91I, PauI and SspI. The plasmid is called pTrc99AcysM (Figure 3).

### 3b) Preparation of L-threonine with the strain MG442/pTrc99AcysM

The L-threonine-producing E. coli strain MG442 is described in the patent specification US-A- 4,278,765 and deposited as CMIM B-1628 at the Russian National Collection for Industrial Microorganisms (VKPM, Moscow, Russia).

The strain MG442 is transformed with the expression plasmid pTrc99AcysM described in example 3a and with the vector pTrc99A and plasmid-carrying cells are selected on LB agar with 50 µg/ml ampicillin. The strains MG442/pTrc99AcysM and MG442/pTrc99A are formed in this manner. Selected individual colonies are then multiplied further on minimal medium with the following composition: 3.5 g/l Na₂HPO₄*2H₂O, 1.5 g/l KH₂PO₄, 1 g/l NH₄Cl, 0.1 g/l MgSO₄*7H₂O, 2 g/l glucose, 20 g/l agar, 50 mg/l ampicillin. The formation of L-threonine is checked in batch cultures of 10 ml contained in 100 ml conical flasks. For this, 10 ml of preculture medium of the following composition: 2 g/l yeast extract, 10 g/l (NH₄)₂SO₄, 1 g/l KH₂PO₄, 0.5 g/l MgSO₄*7H₂O, 15 g/l CaCO₃, 20 g/l glucose, 50 mg/l ampicillin are inoculated and the batch is incubated for 16 hours at 37°C and 180 rpm on an ESR incubator from Kuhner AG (Birsfelden, Switzerland).

250 µl portions of this preculture are transinoculated into 10 ml of production medium (25 g/l (NH₄)₂SO₄, 2 g/l KH₂PO₄, 1 g/l MgSO₄*7H₂O, 0.03 g/l FeSO₄*7H₂O, 0.018 g/l MnSO₄*1H₂O, 30 g/l CaCO₃, 20 g/l glucose, 50 mg/l ampicillin) and the batch is incubated for 48 hours at 37°C. The formation of L-threonine by the starting strain MG442 is investigated in the same manner, but no addition of ampicillin to the medium takes place. After the incubation the optical density (OD) of the culture suspension is determined with an LP2W photometer from Dr. Lange (Dusseldorf, Germany) at a measurement wavelength of 660 nm.

The concentration of L-threonine formed is then determined in the sterile-filtered culture supernatant with an amino acid analyzer from Eppendorf-BioTronik (Hamburg, Germany) by ion exchange chromatography and post-column reaction with ninhydrin detection.

The result of the experiment is shown in Table 3.

**Table 3**

| Strain | OD (660 nm) | L-threonine g/l |
|---|---|---|
| MG442 | 5.6 | 1.4 |
| MG442/pTrc99A | 3.8 | 1.3 |
| MG442/pTrc99AcysM | 1.6 | 2.0 |

### Example 4 (not part of the invention)

### Preparation of L-threonine using the cysP, cysU, cysW and cysA genes

### 4a) Construction of the expression plasmid pTrc99AcysPUWA

The cysP, cysU, cysW and cysA genes from E. coli K12 are amplified using the polymerase chain reaction (PCR) and synthetic oligonucleotides. Starting from the nucleotide sequence of the cysP, cysU, cysW and cysA genes in E. coli K12 MG1655 (Accession Number AE000329 and AE000330, Blattner et al. (Science 277: 1453-1462 (1997)), PCR primers are synthesized (MWG Biotech, Ebersberg, Germany). The sequences of the primers are modified such that recognition sites for restriction enzymes are formed. The recognition sequence for XbaI is chosen for the cysPUWA1 primer and the recognition sequence for HindIII for the cysPUWA2 primer, which are marked by underlining in the nucleotide sequence shown below:
cysPUWA1: 5' - GTCTCTAGATAAATAAGGGTGCGCAATGGC - 3' (SEQ ID No. 7)
cysPUWA2: 5' - CCGGGCGTTTAAGCTTCACTCAACC - 3' (SEQ ID No. 8)

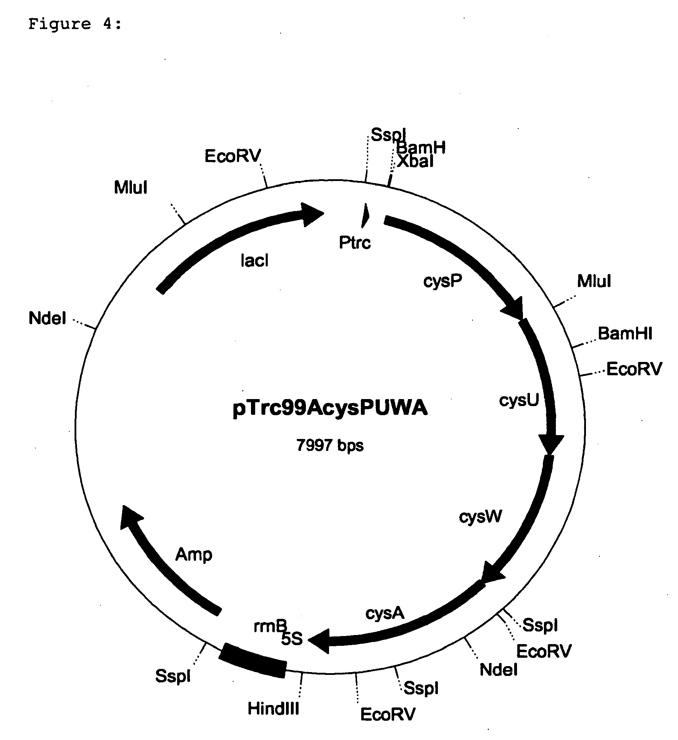

The chromosomal E. coli K12 MG1655 DNA employed for the PCR is isolated according to the manufacturer's instructions with "Qiagen Genomic-tips 100/G" (QIAGEN, Hilden, Germany). A DNA fragment approx. 3900 bp in size can be amplified with the specific primers under standard PCR conditions (Innis et al. (1990) PCR Protocols. A Guide to Methods and Applications, Academic Press) with Pfu-DNA polymerase (Promega Corporation, Madison, USA). The PCR product is cleaved with the restriction enzymes XbaI and HindIII and ligated with the vector pTrc99A (Pharmacia Biotech, Uppsala, Sweden), which has been digested with the enzymes XbaI and HindIII. The E. coli strain XL1-Blue MRF' (Stratagene, La Jolla, USA) is transformed with the ligation batch and plasmid-carrying cells are selected on LB agar, to which 50 µg/ml ampicillin are added. Successful cloning can be demonstrated after plasmid DNA isolation by control cleavage with the enzymes BamHI, EcoRV, MluI, NdeI and SspI. The plasmid is called pTrc99AcysPUWA (Figure 4).

### 4b) Preparation of L-threonine with the strain MG442/pTrc99AcysPUWA

The L-threonine-producing E. coli strain MG442 is described in the patent specification US-A- 4,278,765 and deposited as CMIM B-1628 at the Russian National Collection for Industrial Microorganisms (VKPM, Moscow, Russia).

The strain MG442 is transformed with the expression plasmid pTrc99AcysPUWA described in example 4a and with the vector pTrc99A and plasmid-carrying cells are selected on LB agar with 50 µg/ml ampicillin. The strains MG442/pTrc99AcysPUWA and MG442/pTrc99A are formed in this manner. Selected individual colonies are then multiplied further on minimal medium with the following composition: 3.5 g/l Na₂HPO₄*2H₂O, 1.5 g/l KH₂PO₄, 1 g/l NH₄Cl, 0.1 g/l MgSO₄*7H₂O, 2 g/l glucose, 20 g/l agar, 50 mg/l ampicillin. The formation of L-threonine is checked in batch cultures of 10 ml contained in 100 ml conical flasks. For this, 10 ml of preculture medium of the following composition: 2 g/l yeast extract, 10 g/l (NH₄)₂SO₄, 1 g/l KH₂PO₄, 0.5 g/l MgSO₄*7H₂O, 15 g/l CaCO₃, 20 g/l glucose, 50 mg/l ampicillin are inoculated and the batch is incubated for 16 hours at 37°C and 180 rpm on an ESR incubator from Kühner AG (Birsfelden, Switzerland).

250 µl portions of this preculture are transinoculated into 10 ml of production medium (25 g/l (NH₄)₂SO₄, 2 g/l KH₂PO₄, 1 g/l MgSO₄*7H₂O, 0.03 g/l FeSO₄*7H₂O, 0.018 g/l MnSO₄*1H₂O, 30 g/l CaCO₃, 20 g/l glucose, 50 mg/l ampicillin) and the batch is incubated for 48 hours at 37°C. For complete induction of the expression of the cysPUWA genes, 100 mg/l isopropyl β-D-thiogalactopyranoside (IPTG) are added in parallel batches. The formation of L-threonine by the starting strain MG442 is investigated in the same manner, but no addition of ampicillin to the medium takes place. After the incubation the optical density (OD) of the culture suspension is determined with an LP2W photometer from Dr. Lange (Düsseldorf, Germany) at a measurement wavelength of 660 nm.

The concentration of L-threonine formed is then determined in the sterile-filtered culture supernatant with an amino acid analyzer from Eppendorf-BioTronik (Hamburg, Germany) by ion exchange chromatography and post-column reaction with ninhydrin detection.

The result of the experiment is shown in Table 4.

**Table 4**

| Strain | Additives | OD (660 nm) | L-Threonine g/l |
|---|---|---|---|
| MG442 | - | 5.6 | 1.4 |
| MG442/pTrc99A | - | 3.8 | 1.3 |
| MG442/pTrc99AcysPUWA | - | 5.5 | 1.7 |
| MG442/pTrc99AcysPUWA | IPTG | 6.5 | 2.1 |

### Example 5(not part of the invention)

### Preparation of L-threonine using the cysD, cysN and cysC genes

### 5a) Construction of the expression plasmid pTrc99AcysDNC

The cysD, cysN and cysC genes from E. coli K12 are amplified using the polymerase chain reaction (PCR) and synthetic oligonucleotides. Starting from the nucleotide sequence of the cysD, cysN and cysC genes in E. coli K12 MG1655 (Accession Number AE000358, Blattner et al. (Science 277: 1453-1462 (1997)), PCR primers are synthesized (MWG Biotech, Ebersberg, Germany). The sequences of the primers are modified such that recognition sites for restriction enzymes are formed. The recognition sequence for XbaI is chosen for the cysDNC1 primer and the recognition sequence for HindIII for the cysDNC2 primer, which are marked by underlining in the nucleotide sequence shown below:
cysDNC1: 5' - GCAAGAAAATAGCGGTCTAGATAAGGAACG - 3' (SEQ ID No. 9)
cysDNC2: 5` - CATGGAAAGCTTGTGGTGTCTCAGG - 3' (SEQ ID No. 10)

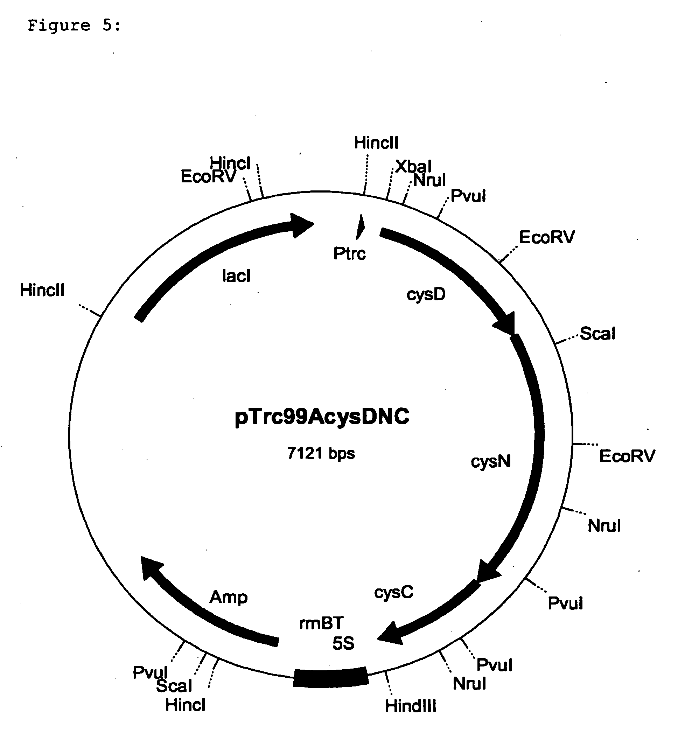

The chromosomal E. coli K12 MG1655 DNA employed for the PCR is isolated according to the manufacturer,s instructions with "Qiagen Genomic-tips 100/G" (QIAGEN, Hilden, Germany). A DNA fragment approx. 3000 bp in size can be amplified with the specific primers under standard PCR conditions (Innis et al. (1990) PCR Protocols. A Guide to Methods and Applications, Academic Press) with Pfu-DNA polymerase (Promega Corporation, Madison, USA). The PCR product is cleaved with the restriction enzymes XbaI and HindIII and ligated with the vector pTrc99A (Pharmacia Biotech, Uppsala, Sweden), which has been digested with the enzymes XbaI and HindIII. The E. coli strain XL1-Blue MRF' (Stratagene, La Jolla, USA) is transformed with the ligation batch and plasmid-carrying cells are selected on LB agar, to which 50 µg/ml ampicillin are added. Successful cloning can be demonstrated after plasmid DNA isolation by control cleavage with the enzymes EcoRV, HincII, NruI, PvuI ans ScaI. The plasmid is called pTrc99AcysDNC (Figure 5).

### 5b) Preparation of L-threonine with the strain MG442/pTrc99AcysDNC

The L-threonine-producing E. coli strain MG442 is described in the patent specification US-A- 4,278,765 and deposited as CMIM B-1628 at the Russian National Collection for Industrial Microorganisms (VKPM, Moscow, Russia).

The strain MG442 is transformed with the expression plasmid pTrc99AcysDNC described in example 5a and with the vector pTrc99A and plasmid-carrying cells are selected on LB agar with 50 µg/ml ampicillin. The strains MG442/pTrc99AcysDNC and MG442/pTrc99A are formed in this manner. Selected individual colonies are then multiplied further on minimal medium with the following composition: 3.5 g/l Na₂HPO₄*2H₂O, 1.5 g/l KH₂PO₄, 1 g/l NH₄Cl, 0.1 g/l MgSO₄*7H₂O, 2 g/l glucose, 20 g/l agar, 50 mg/l ampicillin. The formation of L-threonine is checked in batch cultures of 10 ml contained in 100 ml conical flasks. For this, 10 ml of preculture medium of the following composition: 2 g/l yeast extract, 10 g/l (NH₄)₂SO₄, 1 g/l KH₂PO₄, 0.5 g/l MgSO₄*7H₂O, 15 g/l CaCO₃, 20 g/l glucose, 50 mg/l ampicillin are inoculated and the batch is incubated for 16 hours at 37°C and 180 rpm on an ESR incubator from Kuhner AG (Birsfelden, Switzerland).

250 µl portions of this preculture are transinoculated into 10 ml of production medium (25 g/l (NH₄)₂SO₄, 2 g/l KH₂PO₄, 1 g/l MgSO₄*7H₂O, 0.03 g/l FeSO₄*7H₂O, 0.018 g/l MnSO₄*1H₂O, 30 g/l CaCO₃, 20 g/l glucose, 50 mg/l ampicillin) and the batch is incubated for 48 hours at 37°C. The formation of L-threonine by the starting strain MG442 is investigated in the same manner, but no addition of ampicillin to the medium takes place. After the incubation the optical density (OD) of the culture suspension is determined with an LP2W photometer from Dr. Lange (Dusseldorf, Germany) at a measurement wavelength of 660 nm.

The concentration of L-threonine formed is then determined in the sterile-filtered culture supernatant with an amino acid analyzer from Eppendorf-BioTronik (Hamburg, Germany) by ion exchange chromatography and post-column reaction with ninhydrin detection.

The result of the experiment is shown in Table 5.

**Table 5**

| Strain | OD (660 nm) | L-Threonine g/l |
|---|---|---|
| MG442 | 5.6 | 1.4 |
| MG442/pTrc99A | 3.8 | 1.3 |
| MG442/pTrc99AcysDNC | 5.1 | 2.5 |

### Example 6(not part of the invention)

### Preparation of L-threonine using the cysJ and cysI genes

### 6a) Construction of the expression plasmid pTrc99AcysJI

The cysJ and cysI genes from E. coli K12 are amplified using the polymerase chain reaction (PCR) and synthetic oligonucleotides. Starting from the nucleotide sequence of the cysJ and cysI genes in E. coli K12 MG1655 (Accession Number AE000360, Blattner et al. (Science 277: 1453-1462 (1997)), PCR primers are synthesized (MWG Biotech, Ebersberg, Germany):
cysJI1: 5' - CTGGAACATAACGACGCATGAC - 3' (SEQ ID No. 11)
cysJI2: 5' - GACCGGGCTGATGGTTAATCC - 3' (SEQ ID No. 12)

The chromosomal E. coli K12 MG1655 DNA employed for the PCR is isolated according to the manufacturer,s instructions with "Qiagen Genomic-tips 100/G" (QIAGEN, Hilden, Germany). A DNA fragment approx. 3550 bp in size can be amplified with the specific primers under standard PCR conditions (Innis et al. (1990) PCR Protocols. A Guide to Methods and Applications, Academic Press) with Pfu-DNA polymerase (Promega Corporation, Madison, USA). The PCR product is ligated according to the manufacturer's instructions with the vector pCR-Blunt II-TOPO (Zero Blunt TOPO PCR Cloning Kit, Invitrogen, Groningen, The Netherlands) and transformed into the E. coli strain TOP10.

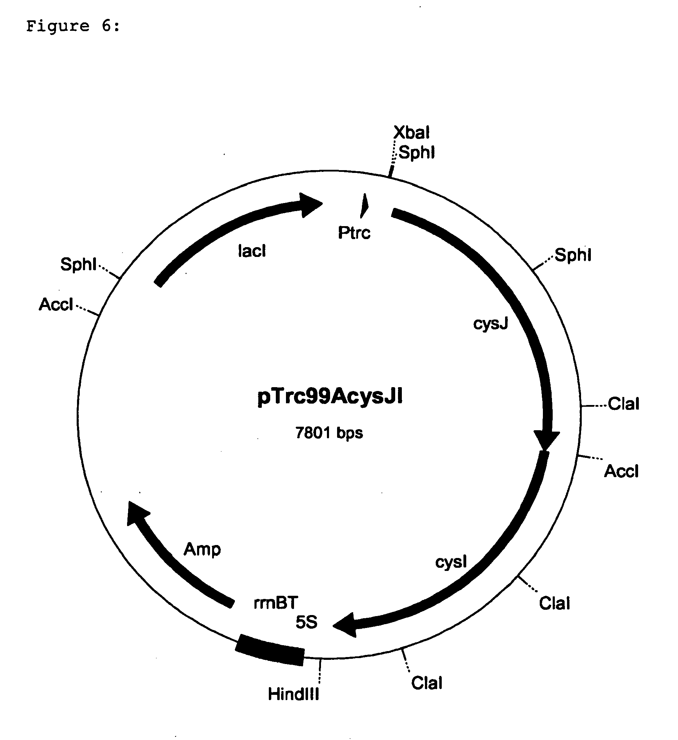

Selection of plasmid-carrying cells takes place on LB agar, to which 50 µg/ml kanamycin are added. After isolation of the plasmid DNA, the vector pCR-Blunt II-TOPO-cysJI is cleaved with the restriction enzymes HindIII and XbaI and, after separation in 0.8% agarose gel, the cysJI fragment is isolated with the aid of the QIAquick Gel Extraction Kit (QIAGEN, Hilden, Germany). The vector pTrc99A (Pharmacia Biotech, Uppsala, Sweden) is cleaved with the enzymes HindIII and XbaI and ligation is carried out with the cysJI fragment isolated. The E. coli strain XL1-Blue MRF' (Stratagene, La Jolla, USA) is transformed with the ligation batch and plasmid-carrying cells are selected on LB agar, to which 50 µg/ml ampicillin are added. Successful cloning can be demonstrated after plasmid DNA isolation by control cleavage with the enzymes AccI, ClaI and SphI. The plasmid is called pTrc99AcysJI (Figure 6).

### 6b) Preparation of L-threonine with the strain MG442/pTrc99AcysJI

The L-threonine-producing E. coli strain MG442 is described in the patent specification US-A- 4,278,765 and deposited as CMIM B-1628 at the Russian National Collection for Industrial Microorganisms (VKPM, Moscow, Russia).

The strain MG442 is transformed with the expression plasmid pTrc99AcysJI described in example 6a and with the vector pTrc99A and plasmid-carrying cells are selected on LB agar with 50 µg/ml ampicillin. The strains MG442/pTrc99AcysJI and MG442/pTrc99A are formed in this manner. Selected individual colonies are then multiplied further on minimal medium with the following composition: 3.5 g/l Na₂HPO₄*2H₂O, 1.5 g/l KH₂PO₄, 1 g/l NH₄Cl, 0.1 g/l MgSO₄*7H₂O, 2 g/l glucose, 20 g/l agar, 50 mg/l ampicillin. The formation of L-threonine is checked in batch cultures of 10 ml contained in 100 ml conical flasks. For this, 10 ml of preculture medium of the following composition: 2 g/l yeast extract, 10 g/l (NH₄)₂SO₄, 1 g/l KH₂PO₄, 0.5 g/l MgSO₄*7H₂O, 15 g/l CaCO₃, 20 g/l glucose, 50 mg/l ampicillin are inoculated and the batch is incubated for 16 hours at 37°C and 180 rpm on an ESR incubator from Kühner AG (Birsfelden, Switzerland).

250 µl portions of this preculture are transinoculated into 10 ml of production medium (25 g/l (NH₄)₂SO₄, 2 g/l KH₂PO₄, 1 g/l MgSO₄*7H₂O, 0.03 g/l FeSO₄*7H₂O, 0.018 g/l MnSO₄*1H₂O, 30 g/l CaCO₃, 20 g/l glucose, 50 mg/l ampicillin) and the batch is incubated for 48 hours at 37°C. The formation of L-threonine by the starting strain MG442 is investigated in the same manner, but no addition of ampicillin to the medium takes place. After the incubation the optical density (OD) of the culture suspension is determined with an LP2W photometer from Dr. Lange (Dusseldorf, Germany) at a measurement wavelength of 660 nm.

The concentration of L-threonine formed is then determined in the sterile-filtered culture supernatant with an amino acid analyzer from Eppendorf-BioTronik (Hamburg, Germany) by ion exchange chromatography and post-column reaction with ninhydrin detection.

The result of the experiment is shown in Table 6.

**Table 6**

| Strain | OD (660 nm) | L-Threonine g/l |
|---|---|---|
| MG442 | 5.6 | 1.4 |
| MG442/pTrc99A | 3.8 | 1.3 |
| MG442/pTrc99AcysJI | 6.3 | 2.5 |

### Example 7(not part of the invention)

### Preparation of L-threonine using the cysH gene

### 7a) Construction of the expression plasmid pTrc99AcysH

The cysH gene from E. coli K12 is amplified using the polymerase chain reaction (PCR) and synthetic oligonucleotides. Starting from the nucleotide sequence of the cysH gene in E. coli K12 MG1655 (Accession Number AE000360, Blattner et al. (Science 277: 1453-1462 (1997)), PCR primers are synthesized (MWG Biotech, Ebersberg, Germany):
cysH1: 5' - GGCAAACAGTGAGGAATCTATG - 3' (SEQ ID No. 13)
cysH2: 5' - GTCCGGCAATATTTACCCTTC - 3' (SEQ ID No. 14)

The chromosomal E. coli K12 MG1655 DNA employed for the PCR is isolated according to the manufacturer,s instructions with "Qiagen Genomic-tips 100/G" (QIAGEN, Hilden, Germany). A DNA fragment approx. 800 bp in size can be amplified with the specific primers under standard PCR conditions (Innis et al. (1990) PCR Protocols. A Guide to Methods and Applications, Academic Press) with Pfu-DNA polymerase (Promega Corporation, Madison, USA). The PCR product is ligated according to the manufacturer's instructions with the vector pCR-Blunt II-TOPO (Zero Blunt TOPO PCR Cloning Kit, Invitrogen, Groningen, The Netherlands) and transformed into the E. coli strain TOP10.

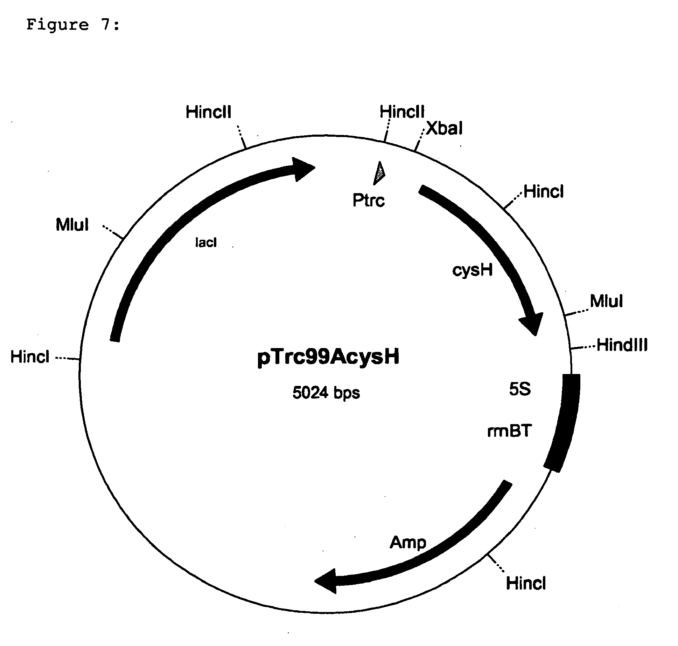

Selection of plasmid-carrying cells takes place on LB agar, to which 50 µg/ml kanamycin are added. After isolation of the plasmid DNA, the vector pCR-Blunt II-TOPO-cysH is cleaved with the restriction enzymes HindIII and XbaI and, after separation in 0.8% agarose gel, the cysH fragment is isolated with the aid of the QIAquick Gel Extraction Kit (QIAGEN, Hilden, Germany). The vector pTrc99A (Pharmacia Biotech, Uppsala, Sweden) is cleaved with the enzymes HindIII and XbaI and ligation is carried out with the cysH fragment isolated. The E. coli strain XL1-Blue MRF' (Stratagene, La Jolla, USA) is transformed with the ligation batch and plasmid-carrying cells are selected on LB agar, to which 50 µg/ml ampicillin are added. Successful cloning can be demonstrated after plasmid DNA isolation by control cleavage with the enzymes HincII and MluI. The plasmid is called pTrc99AcysH (Figure 7).

### 7b) Preparation of L-threonine with the strain MG442/pTrc99AcysH

The L-threonine-producing E. coli strain MG442 is described in the patent specification US-A- 4,278,765 and deposited as CMIM B-1628 at the Russian National Collection for Industrial Microorganisms (VKPM, Moscow, Russia).

The strain MG442 is transformed with the expression plasmid pTrc99AcysH described in example 7a and with the vector pTrc99A and plasmid-carrying cells are selected on LB agar with 50 µg/ml ampicillin. The strains MG442/pTrc99AcysH and MG442/pTrc99A are formed in this manner. Selected individual colonies are then multiplied further on minimal medium with the following composition: 3.5 g/l Na₂HPO₄*2H₂O, 1.5 g/l KH₂PO₄, 1 g/l NH₄Cl, 0.1 g/l MgSO₄*7H₂O, 2 g/l glucose, 20 g/l agar, 50 mg/l ampicillin. The formation of L-threonine is checked in batch cultures of 10 ml contained in 100 ml conical flasks. For this, 10 ml of preculture medium of the following composition: 2 g/l yeast extract, 10 g/l (NH₄)₂SO₄, 1 g/l KH₂PO₄, 0.5 g/l MgSO₄*7H₂O, 15 g/l CaCO₃, 20 g/l glucose, 50 mg/l ampicillin are inoculated and the batch is incubated for 16 hours at 37°C and 180 rpm on an ESR incubator from Kühner AG (Birsfelden, Switzerland). 250 µl portions of this preculture are transinoculated into 10 ml of production medium (25 g/l (NH₄)₂SO₄, 2 g/l KH₂PO₄, 1 g/l MgSO₄*7H₂O, 0.03 g/l FeSO₄*7H₂O, 0.018 g/l MnSO₄*1H₂O, 30 g/l CaCO₃, 20 g/l glucose, 50 mg/l ampicillin) and the batch is incubated for 48 hours at 37°C. The formation of L-threonine by the starting strain MG442 is investigated in the same manner, but no addition of ampicillin to the medium takes place. After the incubation the optical density (OD) of the culture suspension is determined with an LP2W photometer from Dr. Lange (Dusseldorf, Germany) at a measurement wavelength of 660 nm.

The concentration of L-threonine formed is then determined in the sterile-filtered culture supernatant with an amino acid analyzer from Eppendorf-BioTronik (Hamburg, Germany) by ion exchange chromatography and post-column reaction with ninhydrin detection.

The result of the experiment is shown in Table 7.

**Table 7**

| Strain | OD (660 nm) | L-Threonine g/l |
|---|---|---|
| MG442 | 5.6 | 1.4 |
| MG442/pTrc99A | 3.8 | 1.3 |
| MG442/pTrc99AcysH | 4.1 | 2.7 |

### Brief Description of the Figures:

- Figure 1:: Map of the plasmid pTrc99AcysB containing the cysB gene.
- Figure 2:: Map of the plasmid pTrc99AcysK containing the cysK gene.
- Figure 3:: Map of the plasmid pTrc99AcysM containing the cysM gene.
- Figure 4:: Map of the plasmid pTrc99AcysPUWA containing the cysP, cysU, cysW and cysA genes.
- Figure 5:: Map of the plasmid pTrc99AcysDNC containing the cysD, cysN and cysC genes.
- Figure 6:: Map of the plasmid pTrc99AcysJI containing the cysJ and cysI genes.
- Figure 7:: Map of the plasmid pTrc99AcysH containing the cysH gene.

The length data are to be understood as approx. data. The abbreviations and designations used have the following meaning:
- Amp: Ampicillin resistance gene
- lacI: Gene for the repressor protein of the trc promoter
- Ptrc: trc promoter region, IPTG-inducible
- cysB: Coding region of the cysB gene
- cysK: Coding region of the cysK gene
- cysM: Coding region of the cysM gene
- cysP: Coding region of the cysP gene
- cysU: Coding region of the cysU gene
- cysW: Coding region of the cysW gene
- cysA: Coding region of the cysA gene
- cysD: Coding region of the cysD gene
- cysN: Coding region of the cysN gene
- cysC: Coding region of the cysC gene
- cysJ: Coding region of the cysJ gene
- cysI: Coding region of the cysI gene
- cysH: Coding region of the cysH gene
- 5S: 5S rRNA region
- rrnBT: rRNA terminator region

The abbreviations for the restriction enzymes have the following meaning
- AccI: Restriction endonuclease from Acinetobacter calcoaceticus
- BamHI: Restriction endonuclease from Bacillus amyloliquefaciens H
- BstEII: Restriction endonuclease from Bacillus stearothermophilus ATCC 12980
- ClaI: Restriction endonuclease from Caryophannon latum
- EcoRI: Restriction endonuclease from Escherichia coli RY13
- EcoRV: Restriction endonuclease from Escherichia coli B946
- HincII: Restriction endonuclease from Haemophilus influenzae R_{c}
- HindIII: Restriction endonuclease from Haemophilus influenzae
- MluI: Restriction endonuclease from Micrococcus luteus IFO 12992
- NdeI: Restriction endonuclease from Neisseria dentrificans
- NruI: Restriction endonuclease from Norcadia ruba (ATCC 15906)
- PauI: Restriction endonuclease from Paracoccus alcaliphilus
- PvuI: Restriction endonuclease from Proteus vulgaris (ATCC 13315)
- PvuII: Restriction endonuclease from Proteus vulgaris (ATCC 13315)
- ScaI: Restriction endonuclease from Streptomyces caespitosus
- SmaI: Restriction endonuclease from Serratia marcescens
- SpeI: Restriction endonuclease from Sphaerotilus species ATCC 13923
- SphI: Restriction endonuclease from Streptomyces phaeochromogenes
- SspI: Restriction endonuclease from Sphaerotilus species ATCC 13925
- XbaI: Restriction endonuclease from Xanthomonas campestris

## Claims

1. A process for the preparation of L-threonine which comprises carrying out the following steps:
a) fermentation of microorganisms of the Enterobacteriaceae family which produce L-threonine and in which at least the cysK gene of the cysteine biosynthesis pathway, or nucleotide sequences which code for the gene product of said gene, is (are) over-expressed by increasing the number of copies of said gene or nucleotide sequences and/or by using a potent promoter.
b) concentration of L-threonine in the medium or in the cells of the microorganisms, and
c) isolation of L-threonine, constituents of the fermentation broth and/or the biomass in its entirety or portions. (> 0 to 100%) thereof optionally remaining in the product.

2. A process as claimed in claim 1, wherein the polynucleotide (s) which code(s) for one or more of the gene products of cysteine biosynthesis genes chosen from the group consisting of cysG, cysZ, cysM, cysA, cysW, cysU, cysP, cysD, cysN, cysC, cysJ, cysI, cysH, cysE and sbp is (are) over-expressed.

3. A process as claimed in claim 1, wherein, for the preparation of L-threonine, microorganisms of the Enterobacteriaceae family are fermented, in which in addition at the same time one or more of the genes chosen from the group consisting of:
a) the thrABC operon which codes for aspartate kinase, homoserine dehydrogenase, homoserine kinase and threonine synthase,
b) the pyc gene which codes for pyruvate carboxylase,
c) the pps gene which codes for phosphoenol pyruvate synthase,
d) the ppc gene which codes for phosphoenol pyruvate carboxylase,
e) the pntA and pntB genes which code for transhydrogenase,
f) the rhtB gene which imparts homoserine resistance,
g) the mqo gene which codes for malate:quinone oxidoreductase,
h) the rhtC gene which imparts threonine resistance,
i) the thrE gene which codes for the threonine export protein
j) the gdhA gene which codes for glutamate dehydrogenase
k) the hns gene which codes for the DNA-binding protein HLP-II,
l) the pgm gene which codes for phosphoglucomutase,
m) the fba gene which codes for fructose biphosphate aldolase,
n) the ptsH gene which codes for the phosphohistidine protein hexose phosphotransferase,
o) the ptsI gene which codes for enzyme I of the phosphotransferase system,
p) the crr gene which codes for the glucose-specific IIA component,
q) the ptsG gene which codes for the glucose-specific IIBC component,
r) the lrp gene which codes for the regulator of the leucine regulon,
s) the mopB gene which codes for 10 Kd chaperone,
t) the ahpC gene which codes for the small sub-unit of alkyl hydroperoxide reductase,
u) the ahpF gene which codes for the large sub-unit of alkyl hydroperoxide reductase,
is or are over-expressed.

4. A process as claimed in claim 1, wherein, for the preparation of L-threonine or, microorganisms of the Enterobacteriaceae family are fermented, in which in addition at the same time one or more of the genes chosen from the group consisting of:
a) the tdh gene which codes for threonine dehydrogenase,
b) the mdh gene which codes for malate dehydrogenase,
c) the gene product of the open reading frame (orf) yjfA,
d) the gene product of the open reading frame (orf) yjfP,
e) the pckA gene which codes for phosphoenol pyruvate carboxykinase,
f) the poxB gene which codes for pyruvate oxidase,
g) the aceA gene which codes for isocitrate lyase,
h) the dgsA gene which codes for the DgsA regulator of the phosphotransferase system,
i) the fruR gene which codes for the fructose repressor,
j) the rpoS gene which codes for the sigma³⁸ factor
is or are eliminated.

5. L-Threonine producing microorganisms of the enterobacteriaceae family, wherein at least the thrABC operon, which genes code for aspartate kinase, homoserine dehydrogenase, homoserine kinase and threonine synthase, and the cysK gene is over-expressed, by increasing the number of copies of said genes or nucleotide sequences and/or by using a potent promoter.

6. Microorganisms according to claim 5, wherein the microorganisms originate from the genus Escherichia.

7. Microorganisms according to claim 6, wherein the microorganisms originate from the species E. coli.

## Patentansprüche

1. Verfahren zur Herstellung von L-Threonin, bei dem man folgende Schritte durchführt:
a) Fermentation von L-Threonin produzierenden Mikroorganismen der Familie Enterobacteriaceae, in denen man zumindest das cysK-Gen der Cystein-Biosynthese oder für das Genprodukt des genannten Gens kodierende Nukleotidsequenzen durch Erhöhen der Kopienzahl des genannten Gens beziehungsweise der genannten Nukleotidsequenzen und/oder durch Verwendung eines starken Promotors überexprimiert,
b) Anreicherung des L-Threonins im Medium oder in den Zellen der Mikroorganismen und
c) Isolierung des L-Threonins, wobei gegebenenfalls Bestandteile der Fermentationsbrühe und/oder die Biomasse in ihrer Gesamtheit oder Anteile (> 0 bis 100%) davon im Produkt verbleiben.

2. Verfahren nach Anspruch 1, bei dem man das beziehungsweise die für eines oder mehrere der Genprodukte der Cystein-Biosynthese-Gene, ausgewählt aus der Gruppe cysG, cysZ, cysM, cysA, cysW, cysU, cysP, cysD, cysN, cysC, cysJ, cysI, cysH, cysE und sbp, kodierende(n) Polynukleotid(e) überexprimiert.

3. Verfahren nach Anspruch 1, bei dem man zur Herstellung von L-Threonin Mikroorganismen der Familie Enterobacteriaceae fermentiert, in denen man zusätzlich eines oder mehrere der Gene, ausgewählt aus der Gruppe:
a) das für die Aspartatkinase, die Homoserin-Dehydrogenase, die Homoserinkinase und die Threoninsynthase kodierende thrABC-Operon,
b) das für die Pyruvat-Carboxylase kodierende pyc-Gen,
c) das für die Phosphoenolpyruvat-Synthase kodierende pps-Gen,
d) das für die Phosphoenolpyruvat-Carboxylase kodierende ppc-Gen,
e) die für die Transhydrogenase kodierenden Gene pntA und pntB,
f) das Homoserinresistenz vermittelnde Gen rhtB,
g) das für die Malat: Chinon Oxidoreduktase kodierende mqo-Gen,
h) das Threoninresistenz vermittelnde Gen rhtC,
i) das für das Threoninexportprotein kodierende thrE-Gen
j) das für die Glutamat-Dehydrogenase kodierende gdhA-Gen,
k) das für das DNA-Bindeprotein HLP-II kodierende hns-Gen,
l) das für die Phosphoglukomutase kodierende pgm-Gen,
m) das für die Fructose-Biphosphat-Aldolase kodierende fba-Gen,
n) das für die Phosphohistidin-Protein-Hexose-Phosphotransferase kodierende ptsH-Gen,
o) das für Enzym I des Phosphotransferase-Systems kodierende ptsI-Gen,
p) das für die glukosespezifische IIA-Komponente kodierende crr-Gen,
q) das für die glukosespezifische IIBC-Komponente kodierende ptsG-Gen,
r) das für den Regulator des Leucin-Regulons kodierende lrp-Gen,
s) das für 10-Kd-Chaperon kodierende mopB-Gen,
t) das für die kleine Untereinheit der Alkylhydroperoxidreduktase kodierende ahpC-Gen,
u) das für die große Untereinheit der Alkylhydroperoxidreduktase kodierende ahpF-Gen
überexprimiert.

4. Verfahren nach Anspruch 1, bei dem man zur Herstellung von L-Threonin Mikroorganismen der Familie Enterobacteriaceae fermentiert, in denen man zusätzlich gleichzeitig eines oder mehrere der Gene, ausgewählt aus der Gruppe:
a) das für die Threonin-Dehydrogenase kodierende tdh-Gen,
b) das für die Malat-Dehydrogenase kodierende mdh-Gen,
c) das Genprodukt des offenen Leserahmens (orf) yjfA,
d) das Genprodukt des offenen Leserahmens (orf) yjfP,
e) das für die Phosphoenolpyruvat-Carboxykinase kodierende pckA-Gen,
f) das für die Pyruvat-Oxidase kodierende poxB-Gen,
g) das für die Isocitrat-Lyase kodierende aceA-Gen,
h) das für den DgsA-Regulator des Phosphotransferase-Systems kodierende dgsA-Gen,
i) das für den Fructose-Repressor kodierende fruR-Gen,
j) das für den Faktor sigma³⁸ kodierende rpoS-Gen ausschaltet.

5. L-Threonin produzierende Mikroorganismen der Familie Enterobacteriaceae, in denen zumindest das für die Aspartatkinase, Homoserin-Dehydrogenase, Homoserinkinase und Threoninsynthase kodierende thrABC-Operon und das cysK-Gen durch Erhöhen der Kopienzahl der genannten Gene oder Nukleotidsequenzen und/oder durch Verwendung eines starken Promotors überexprimiert sind.

6. Mikroorganismen nach Anspruch 5 der Gattung Escherichia.

7. Mikroorganismen nach Anspruch 6 der Art E. coli.

## Revendications

1. Procédé de préparation de L-thréonine, comprenant la réalisation des étapes suivantes :
a) la fermentation de micro-organismes de la famille des Entérobactéries qui produisent de la L-thréonine et dans lesquels au moins le gène cysK de la voie de biosynthèse de la cystéine, ou les séquences nucléotidiques codant pour le produit génétique dudit gène, est (sont) surexprimé(s) en augmentant le nombre de copies dudit gène ou desdites séquences nucléotidiques et/ou en utilisant un promoteur puissant,
b) la concentration de la L-thréonine dans le milieu ou dans les cellules des micro-organismes, et
c) l'isolement de la L-thréonine, des constituants du bouillon de fermentation et/ou de la biomasse dans son ensemble ou en des portions (> 0 à 100%) de celle-ci restant éventuellement dans le produit.

2. Procédé selon la revendication 1, **caractérisé en ce que** le ou les polynucléotide(s) codant pour un ou plusieurs des produits génétiques des gènes de biosynthèse de la cystéine choisis parmi le groupe constitué par cysG, cysZ, cysM, cysA, cysW, cysU, cysP, cysD, cysN, cysC, cysJ, cysI, cysH, cysE et sbp est (sont) surexprimé(s).

3. Procédé selon la revendication 1, **caractérisé en ce que**, pour la préparation de la L-thréonine, des micro-organismes de la famille des Entérobactéries sont fermentés, dans lesquels outre en même temps, un ou plusieurs des gènes choisis parmi le groupe constitué par :
a) l'opéron thrABC qui code pour l'aspartate kinase, l'homosérine déshydrogénase, l'homosérine kinase et la thréonine synthétase,
b) le gène pyc qui code pour la pyruvate carboxylase,
c) le gène pps qui code pour la phosphoénol pyruvate synthétase,
d) le gène ppc qui code pour la phosphoénol pyruvate carboxylase,
e) les gènes pntA et pntB qui codent pour la transhydrogénase,
f) le gène rhtB qui confère une résistance à l'homosérine,
g) le gène mqo qui code pour la malate:quinone oxydoréductase,
h) le gène rhtC qui confère une résistance à la thréonine,
i) le gène thrE qui code pour la protéine d'exportation de thréonine,
j) le gène gdhA qui code pour la glutamate déshydrogénase,
k) le gène hns qui code pour la protéine HLP-II fixant l'ADN,
l) le gène pgm qui code pour la phosphoglucomutase,
m) le gène fba qui code pour la fructose biphosphate aldolase,
n) le gène ptsH qui code pour la phosphohistidine protéine hexose phosphotransférase,
o) le gène ptsI qui code pour l'enzyme I du système de phosphotransférase,
p) le gène crr qui code pour le composant IIA spécifique du glucose,
q) le gène ptsG qui code pour le composant IIBC spécifique du glucose,
r) le gène lrp qui code pour le régulateur du régulon de la leucine,
s) le gène mopB qui code pour la chaperone de 10 kD,
t) le gène ahpC qui code pour la petite sous-unité de l'alkyl hydroperoxyde réductase,
u) le gène ahpF qui code pour la grande sous-unité de l'alkyl hydroperoxyde réductase,
est (sont) surexprimé(s).

4. Procédé selon la revendication 1, **caractérisé en ce que,** pour la préparation de la L-thréonine, des micro-organismes de la famille des Entérobactéries sont fermentés, dans lesquels outre en même temps, un ou plusieurs des gènes choisis parmi le groupe constitué par :
a) le gène tdh qui code pour la thréonine déshydrogénase,
b) le gène mdh qui code pour la malate déshydrogénase,
c) le produit génétique du cadre ouvert de lecture (orf) yjfA,
d) le produit génétique du cadre ouvert de lecture (orf) yjfP,
e) le gène pckA qui code pour la phosphoénol pyruvate carboxykinase,
f) le gène poxB qui code pour la pyruvate oxydase,
g) le gène aceA qui code pour l'isocitrate lyase,
h) le gène dgsA qui code pour le régulateur DgsA du système de phosphotransférase,
i) le gène fruR qui code pour le répresseur de fructose,
j) le gène rpoS qui code pour le facteur sigma³⁸,
est ou sont éliminé(s).

5. Micro-organismes producteurs de L-thréonine de la famille des Entérobactéries, **caractérisés en ce qu'**au moins l'opéron thrABC, dont les gènes codent pour l'aspartate kinase, l'homosérine déshydrogénase, l'homosérine kinase et la thréonine synthétase, et le gène cysK sont surexprimés, en augmentant le nombre de copies desdits gènes ou desdites séquences nucléotidiques et/ou en utilisant un promoteur puissant.

6. Micro-organismes selon la revendication 5, **caractérisés en ce que** les micro-organismes proviennent du genre Escherichia.

7. Micro-organismes selon la revendication 6, **caractérisés en ce que** les micro-organismes proviennent de l'espèce E. coli.


### SEQUENCE PROTOCOL

<110> Degussa AG
<120> Process for the preparation of L-amino acids using strains of the Enterobacteriaceae family
<130> 010275 BT
<160> 14
<170> PatentIn version 3.1
<210> 1<211> 22<212> DNA<213> artificial sequence
<220><221> Primer<222> (1)..(22)<223> cysB1
<400> 1
   gcgtctaagt ggatggttta ac 22
<210> 2<211> 20<212> DNA<213> artificial sequence
<220><221> Primer<222> (1)..(20)<223> cysB2
<400> 2
   ggtgccgaaa ataacgcaag 20
<210> 3<211> 21<212> DNA<213> artificial sequence
<220><221> Primer<222> (1)..(21)<223> cysK1
<400> 3
   cagttaagga caggccatga g 21
<210> 4<211> 22<212> DNA<213> artificial sequence
<220><221> Primer<222> (1)..(22)<223> cysK2
<400> 4
   gctggcatta ctgttgcaat tc 22
<210> 5<211> 28<212> DNA<213> artificial sequence
<220><221> Primer<222> (1)..(28)<223> cysM1
<400> 5
   cgcatcagtc tagaccacgt taggatag 28
<210> 6<211> 25<212> DNA<213> artificial sequence
<220><221> Primer<222> (1)..(25)<223> cysM2
<400> 6
   catcagtctc cgaagctttt aatcc 25
<210> 7<211> 30<212> DNA<213> artificial sequence
<220><221> Primer<222> (1)..(30)<223> cysPUWA1
<400> 7
   gtctctagat aaataagggt gcgcaatggc 30
<210> 8<211> 25<212> DNA<213> artificial sequence
<220><221> Primer<222> (1)..(25)<223> cysPUWA2
<400> 8
   ccgggcgttt aagcttcact caacc 25
<210> 9<211> 30<212> DNA<213> artificial sequence
<220><221> Primer<222> (1)..(30)<223> cysDNC1
<400> 9
   gcaagaaaat agcggtctag ataaggaacg 30
<210> 10<211> 25<212> DNA<213> artificial sequence
<220><221> Primer<222> (1)..(25)<223> cysDNC2
<400> 10
   catggaaagc ttgtggtgtc tcagg 25
<210> 11<211> 22<212> DNA<213> artificial sequence
<220><221> Primer<222> (1)..(22)<223> cysJI1
<400> 11
   ctggaacata acgacgcatg ac 22
<210> 12<211> 21<212> DNA<213> artificial sequence
<220><221> cysJI2<222> (1)..(21)<223> cysJI2
<400> 12
   gaccgggctg atggttaatc c 21
<210> 13<211> 22<212> DNA<213> artificial sequence
<220><221> Primer<222> (1)..(22)<223> cysH1
<400> 13
   ggcaaacagt gaggaatcta tg 22
<210> 14<211> 21<212> DNA<213> artificial sequence
<220><221> Primer<222> (1)..(21)<223> cysH2
<400> 14
   gtccggcaat atttaccctt c 21